# EUROPÄISCHE PATENTSCHRIFT

(11) **EP 0 728 140 B1**
(45) Veröffentlichungstag und Bekanntmachung des Hinweises auf die Patenterteilung: **19.09.2001**
(21) Anmeldenummer: 95900069.6
(22) Anmeldetag: 08.11.1994
(51) Int. Cl.: C07J 31/00, A61K 31/56, C07J 51/00, C07J 1/00, C07J 41/00

(54) **NEUE STEROIDE MIT RADIKOPHILEN SUBSTITUENTEN, VERFAHREN ZU IHRER HERSTELLUNG UND DIESE VERBINDUNGEN ENTHALTENDE ARZNEIMITTEL**
NEW STEROIDS WITH RADICAL-ATTRACTING AROMATIC SUBSTITUENTS, PROCESS FOR THE PRODUCTION THEREOF AND PHARMACEUTICAL COMPOUNDS CONTAINING THE SAID SUBSTANCES
NOUVEAUX STEROIDES AVEC SUBSTITUANTS A PROPRIETES DE CAPTURE DE RADICAUX, PROCEDE POUR LEUR FABRICATION ET PRODUITS PHARMACEUTIQUES RENFERMANT CES COMPOSES

(30) Priorität: 10.11.1993 DE 4338316
(43) Veröffentlichungstag der Anmeldung: 28.08.1996
(73) Patentinhaber: Jenapharm GmbH & Co. KG, 07745 Jena (DE)
(72) Erfinder: DROESCHER, Peter, D-99423 Weimar (DE); MENZENBACH, Bernd, D-07745 Jena (DE); PONSOLD, Kurt, D-07743 Jena (DE); UNDEUTSCH, Bernd, D-07743 Jena (DE); OETTEL, Michael, D-07743 Jena (DE); RÖMER, Wolfgang, D-07745 Jena (DE); KAUFMANN, Günter, D-07743 Jena (DE); SCHRÖDER, Jens, D-07747 Jena (DE)
(74) Vertreter: Cramer, Eva-Maria
(86) Internationale Anmeldenummer: DE9401310
(87) Internationale Veröffentlichungsnummer: WO9513287

(56) Entgegenhaltungen:
- EP-A- 0 436 936
- WO-A-94/24146
- DE-A- 2 247 390
- GB-A- 1 076 962
- GB-A- 2 008 119
- US-A- 3 193 564
- STEROIDS: STRUCTURE, FUNCTION, AND REGULATION, Bd.56, Nr.7, Juli 1991, STONEHAM, MA US Seiten 375 - 387 M. SALMAN ET AL '17-alpha-Substituted analogs of estradiol for the development of fluorescent estrogen receptor ligands'
- JOURNAL OF PHARMACEUTICAL SCIENCES, Bd.73, Nr.3, März 1984, WASHINGTON US Seiten 416 - 418 S. A. SADEK ET AL 'Estrogenic and Antiestrogenic Activity of Novel Selenosteroids'
- JOURNAL OF MEDICINAL CHEMISTRY, Bd.32, Nr.7, Juli 1989, WASHINGTON US Seiten 1642 - 1652 R. H. PETERS ET AL '17-Desoxy Estrogen Analogs'
- JOURNAL OF MEDICINAL CHEMISTRY, Bd.20, Nr.8, August 1977, WASHINGTON US Seiten 1096 - 1099 E. R. CLARK ET AL 'Potential Steroidal Antiestrogens'
- CHEMICAL ABSTRACTS, vol. 106, no. 23, 8. Juni 1987, Columbus, Ohio, US; abstract no. 196665, I. SHARMA ET AL 'Studies in Antifertility Agents. Part 51. Synthesis and Biological Activity of 6-Arylestradiol Derivatives' Seite 769 ;Spalte 1 ; & INDIAN J. CHEM., SECT. B, Bd.25B, Nr.6, 1986 Seiten 634 - 637
- CHEMICAL ABSTRACTS, vol. 117, no. 11, 14. September 1992, Columbus, Ohio, US; abstract no. 111879, D. VICHARD ET AL 'Controlled and Selective Introduction of a Cp*Ru+ (Cp* = C5Me5) fragment to the 17-alpha-Substituent of 17-alpha-Phenylestradiol' Seite 898 ;Spalte 1 ; & ORGANOMETALLICS, Bd.11, Nr.8, 1992 Seiten 2952 - 2956
- JOURNAL OF THE CHEMICAL SOCIETY, PERKIN TRANSACTIONS 1, Nr.8, 1974, LETCHWORTH GB Seiten 897 - 903 C. L. HEWETT ET AL '18-Norandrosta-8,11,13-trienes. Part II. Aromatisation of 18-Norandrost-13-enes by Bromination and Dehydrobromination'
- CHEMICAL ABSTRACTS, vol. 98, no. 11, 14. März 1983, Columbus, Ohio, US; abstract no. 88908, G. L. MAY ET AL 'The Chemistry of Aryllead(IV)tricarboxylates. Reaction with Enamines' Seite 514 ;Spalte 2 ; & AUST. J. CHEM., Bd.35, Nr.9, 1982 Seiten 1859 - 1871 'CHEMICAL ABSTRACTS ELEVENTH COLLECTIVE FORMULA INDEX, VOLUMES 96-105, 1982-1986' , CHEMICAL ABSTRACTS , COLUMBUS OHIO, US
- CHEMICAL ABSTRACTS, vol. 81, no. 23, 9. Dezember 1974, Columbus, Ohio, US; abstract no. 146058, P. V. SERGEEV ET AL Seite 48 ;Spalte 2 ; & VOPR. MED. KHIM., Bd.20, Nr.4, 1974 Seiten 359 - 362
- CHEMICAL ABSTRACTS, vol. 82, no. 5, 3. Februar 1975, Columbus, Ohio, US; abstract no. 26304, V. M. GUKASOV ET AL Seite 84 ;Spalte 2 ; & BIOFIZIKA, Bd.19, Nr.4, 1974 Seiten 763 - 764
- BIOCHEM. PHARMACOL., Bd.37, Nr.20, 1988 Seiten 3853 - 3860 J. BRAUGHLER ET AL 'Novel Membrane Localized Iron Chelators as Inhibitors of Iron-Dependent Lipid Peroxidation'
- JOURNAL OF MEDICINAL CHEMISTRY, Bd.37, Nr.9, 29. April 1994, WASHINGTON US Seiten 1312 - 1319 M. NUMAZAWA ET AL '6-Alkyl- and 6-Arylandrost-4-ene-3,17-diones as Aromatase Inhibitors. Synthesis and Structure Activity Relationships'
- W.RÖMER et al. Steroids 62, 304 (1997)

## Beschreibung

Die Erfindung betrifft neue Steroide mit radikophilen aromatischen Substituenten, Verfahren zu ihrer Herstellung und diese Verbindungen enthaltende Arzneimittel zur Prophylaxe und Therapie radikalvermittelter Zellschädigungen.

Aus der Fach- und Patentliteratur ist bekannt, daß reaktive Sauerstoffspezies (ROS), freie Sauerstoffradikale und weitere Radikalformen eine wichtige Rolle bei der Entstehung vielfältiger Zellschädigungen spielen, beispielsweise bei ischämischen und traumatischen Organverletzungen, Entzündungs- und Vergiftungsprozessen.
Auch bei Hirn- und Wirbelsäulenverletzungen, Schockzuständen, Schlaganfall, Muskeldystrophie, Emphysemen, ARDS, Asthma, Alterungsprozessen, bei Gewebeschädigungen nach Myokardinfarkt, Vergiftungs- und Verstrahlungsschäden, Verbrennungen und transplantationsbedingten Immunreaktionen ist ein negativer Einfluß von ROS, freien Sauerstoffradikalen und anderen Radikalformen zu verzeichnen. Dabei ist u.a. die Lipidperoxidation und die Oxidation von Low Density Lipoprotein (LDL)-Cholesterol in Verbindung mit irreversiblen Membran- und Endothelschädigungen Ausgangspunkt für solche radikalvermittelten Zeltschädigungen.

Es ist weiterhin bekannt, daß lipophile Substanzen, wie z.B. lipophile Steroide mit Radikalscavengereigenschaften ("radikalfangenden" Eigenschaften) sich zur Prophylaxe und Therapie radikalvermittelter Zellschädigungen eignen können.
Im Unterschied zu den bekannten niedermolekularen phenolischen Antioxidanzien werden diese lipophilen Steroide mit gewisser Selektivität in die Region der Zellmembran transportiert und können dort ihre Wirksamkeit entfalten
Der therapeutische Nutzen wird dabei vom Wirkungsspektrum der jeweiligen Substanz bestimmt.

WO-PS 87/01706; WO-PS 91/11453; EP-PS 0389 368;

EP-PS 0389 369; EP-PS 0389 370 und FR-PS 2 640 977 beschreiben beispielsweise Steroide mit Scavengereigenschaften.
WO-PS 87/01706; WO-PS 91/11453; EP-PS 0389 368/...369/...370 beschreiben Steroide, die am terminalen Kohlenstoff-Atom der C-17-Seitenkette eine Aminogruppe enthalten, die substituiert bzw. Bestandteil eines heterocyclischen Ringsystems sein kann.
In FR-PS 2 640 977 wird ein Strukturtyp aufgezeigt, der am C-11-Atom in β-Position einen substituierten Phenylring aufweist.

In J. Phys. Org. Chem. 3 (1990), 309-315 wird dargestellt, daß Estrogene, speziell Catecholestrogene, als Radikalfänger agieren können. 17β-Estradiol, Estron, Estriol und 2-Hydroxy-estradiol hemmen die Peroxidation in vitro und in vivo.

Die EP-A-436936 beschreibt die allgemeine Verknüpfung von lipophilen Komponenten über ein Brückenglied mit einer antioxidativen Komponente.
Die so charakterisierten Verbindungen dienen dem Schutz lipidhaltiger Substanzen gegen Oxidation und zur Prophylaxe und Therapie von Krankheiten, bei denen Radikale involviert sind.
Als antioxidative Komponenten werden u.a. von Biomolekülen abgeleitete Reste, auch alkylsubstituierte Mono-, Di- und Trihydroxybenzenreste aufgezeigt.
Als lipophile Komponenten sind Cholanderivate, einschließlich des Cholestans, mit C-3 oder dem letzten C der Cholanseitenkette als Verknüpfungsstelle sowie nichtsteroidale Alkyl-, Zykloalkyl- und Fettsäurederivatreste aufgezeigt.

WO-94-24146 beschreibt Androgene, Androgenanaloga mit mindestens einem Substituenten in C6, C11 oder C17-Positionen, der Radikalfängereigenschaften aufweist.

In DE-2247390-A werden 1-Alkylthioandrostan-Derivate mit anaboler und/oder androgener Wirkung und antiartheriosklerotischen Eigenschaften beschrieben, die sich in einem direkten Schutz der Gefäßwand äußern. Die beschriebenen Verbindungen unterscheiden sich sehr stark von den erfindungsgemäßen Verbindungen dadurch, daß sie keinen aromatischen A-Ring und/oder zusätzliche Doppelbindungen in den anderen Ringen enthalten.

Natürliche Steroide, deren einfache Derivate, wie Ester und Ether sowie Diethylstilbestrol als synthetisches nichtsteroidales Estrogen werden in Vopr. Med. Khim. 20 (1974), 359-362 in Bezug auf ihre Hemmung der Lipidperoxidation untersucht. Die untersuchten Verbindungen weisen eine moderate aber unterschiedlich starke Hemmung der Lipidperoxidation auf.

Der Erfindung liegt die Aufgabe zugrunde, neue Steroide mit radikophilen aromatischen Substituenten sowie Verfahren zu ihrer Herstellung zu finden.

Eine weitere Aufgabe der Erfindung ist es, Arzneimittel, die diese Verbindungen enthalten, aufzuzeigen.

Die Aufgabe wird erfindungsgemäß dadurch gelöst, daß neue Steroide mit radikophilen aromatischen Substituenten der allgemeinen Formel I im Anspruch 1 gefunden wurden.
Der radikophile aromatische Substituent Y läßt sich als Bestandteil eines Substituenten der allgemeinen Formeln
-(CH₂)_{0.1} X oder -(CH₂)₀₋₅ Y darstellen, mit X = Y, OY, SY, SeY, NHY,
und 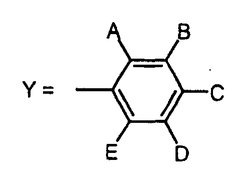 worin, unter der Bedingung, daß mindestens einer der Substituenten A, B, C, D, E eine Hydroxygruppe ist, A bis E unabhängig voneinander H, Alkyl, OAlkyl, OAcyl, OH oder einer der Substituenten B,C,D = NR₂, mit R = Alkyl und den jeweils vier anderen Substituenten = Wasserstoff ist.

Die am meisten bevorzugten Verbindungen der erfindungsgemäßen Steroide mit radikophilen aromatischen Substituenten sind die im Anspruch 2 aufgeführten Verbindungen.

Die Erfindung betrifft weiterhin ein Verfahren zur Herstellung der Steroide nach der allgemeinen Formel I, welches dadurch gekennzeichnet ist, daß die Einführung der radikophilen Substituenten in Position 17 des Steroidgerüsts über die nucleophile Öffnung eines 17-Spirooxirans oder direkt durch Alkylierung der 17-Oxogruppe erfolgt und daß man die direkte Alkylierung der 17-Oxogruppe über die Umsetzung entsprechender Metallorganyle mit Cer-III-Salzen durchführt. Die bei der Herstellung der erfindungsgemäßen Steroide verwendeten Synthesemethoden gehen in der Regel von gut bekannten Steroiden mit dem Estrangrundgerüst aus, in welchen gegebenfalls störende funktionelle Gruppen, z. B. Hydroxy- und Oxogruppen, mit bekannten Methoden zwischenzeitlich, z.B. als Ester, Ether, Silylether, Enolether oder Acetale, geschützt werden.

Die Einführung der radikophilen Substituenten in Position 17 des Steroidgerüstes erfolgt über die nucleophile Öffnung eines 17-Spirooxirans oder direkt durch Alkylierung der 17-Oxogruppe.
Aufgrund der sterischen Hinderung am C-17 gelingt die Einführung der aromatischen Substituenten Y für n = 0 erst durch Umsetzung des 17-Ketons mit der entsprechenden Grignard-Verbindung in Gegenwart von Ce³⁺ unterhalb Raumtemperatur, in einem dipolar-aprotischen Lösungsmittel, vorzugsweise THF oder Diglyme.
Diese Verfahrensweise ist für Steroide neu.
Aus der Literatur ist jedoch bekannt, daß sich Grignardverbindungen in Gegenwart äquimolarer Mengen von Ce-III-Salzen gut zur Alkylierung leicht enolisierbarer oder sterisch behinderter Ketone eignen.

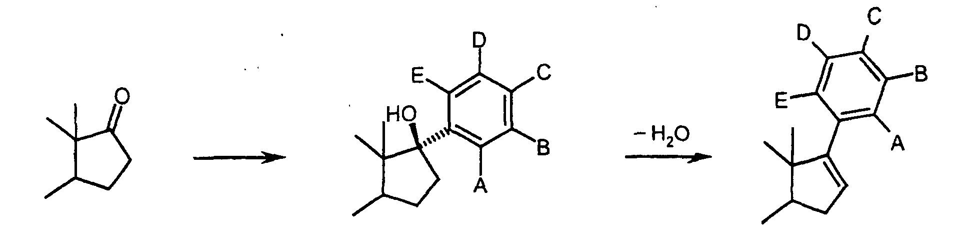

Durch Abspaltung der tertiären benzylischen Hydroxygruppe im sauren Milieu werden die entsprechenden 16,17-ungesättigten Verbindungen erhalten.
17-Spirooxirane werden mit nucleophilen Agenzien unter neutralen oder alkalischen Bedingungen in organischen Lösungsmitteln bei Raumtemperatur oder erhöhter Temperatur zu entsprechenden 17α-substituierten 17-Olen umgesetzt.

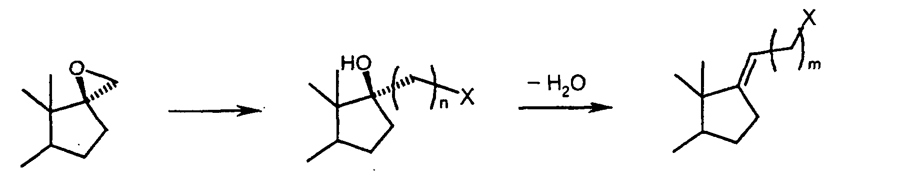

Mittels allgemein bekannter Methoden zur Abspaltung tertiärer Hydroxygruppen, z.B. Erwärmen in hinreichend saurem Milieu, Behandlung des entsprechenden Mesylats mit einer Base oder thermischer Dehydratisierung in DMSO kann man auch zu entsprechenden Olefinen gelangen.

Zur Synthese der 17-Spirooxirane werden die 17-Ketone, z.B. der nachfolgend aufgeführten Steroide mit Dimethylsulfoniummethylid in dipolar-aprotischen Lösungsmitteln oder deren Gemischen in Analogie zu der von Ponsold et al. beschriebenen Methode - Z. Chem. 11; 1972; S.106 - umgesetzt.

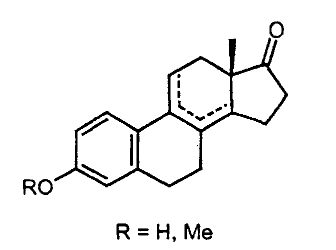

Als nukleophile Agenzien zur Spaltung der 17-Spirooxirane oben aufgeführter Strukturen kommen substituierte Phenolate und Thiophenolate, sowie geeignete metallorganische Reagenzien wie Grignard-Verbindungen und deren O-,S-, und Se-Analoga in Betracht.

Die Wahl des Lösungsmittels bei der Umsetzung der Spirooxirane mit den Phenolaten und Thiophenolaten hängt neben ihrer Eignung für SN₂-Reaktionen auch von den Löslichkeiten der betreffenden Oxirane und des verwendeten nucleophilen Agens ab und kann in weiten Grenzen variiert werden.
Gut geeignet sind aprotische Lösungsmittel, wie Ether, DMSO oder DMF.
Bei Reaktionen mit metallorganischen Reagenzien sind Ether, vorzugsweise cyclische Ether, am geeignetsten.

Silyletherfunktionen, die zur vorübergehenden Maskierung der phenolischen Hydroxygruppen bei Reaktionen mit metallorganischen Reagenzien dienen, werden nach den in der Literatur üblichen Methoden wieder aufgehoben.

Zur Einführung des radikophilen Substituenten in Position 6 von Steroiden mit aromatischem Ring A wird bevorzugt das entsprechende 6-Oxosteroid mit der Grignard-Verbindung in Gegenwart von Ce³⁺ bei Temperaturen unterhalb Raumtemperatur in dipolar-aprotischen Lösungsmitteln, vorzugsweise THF oder Diglyme, umgesetzt.
Durch die Verwendung der intermediär entstehenden, im Vergleich zu den Grignard-Verbindungen niedrigere Basizität besitzenden Cer-Verbindungen wird die Enolisierung des 6-Ketons wirksam zurückgedrängt.

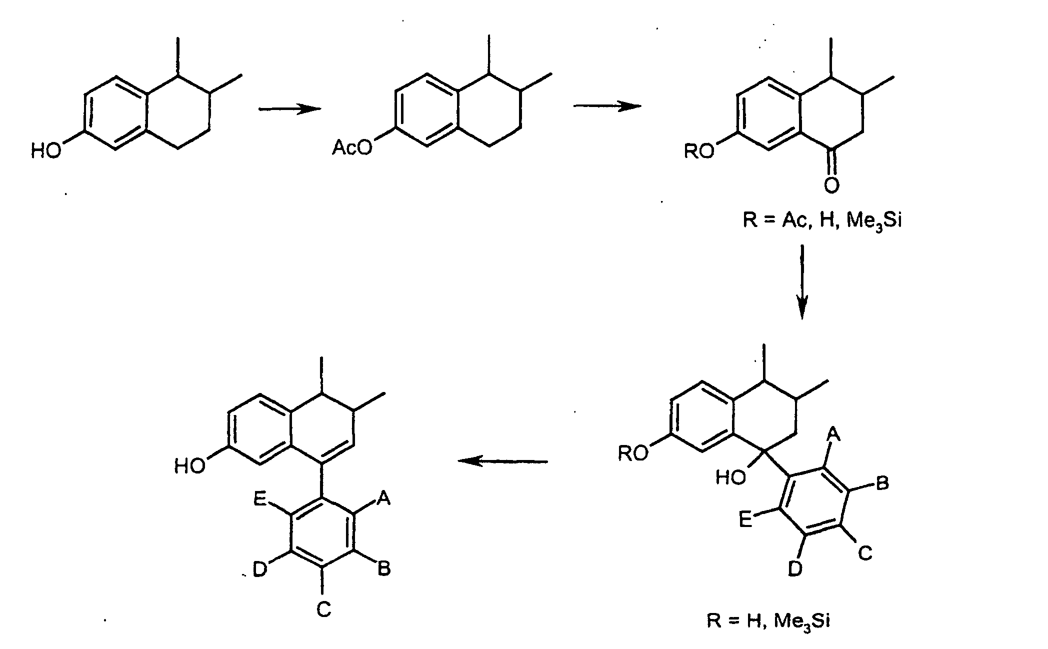

Zum Beispiel durch Behandlung mit Säuren in organischen Lösungsmitteln bei Raumtemperatur kann Wasser abgespalten und die 6-Dehydro-Verbindung erhalten werden.
Die Herstellung des als Ausgangsmaterial dienenden 6-Oxosteroids kann durch verschiedene bekannte Oxidationsmethoden erfolgen, z.B. mit Chrom-Vl-oxid/Dimethylpyrazol in Methylenchlorid.

Selenoaryle können nach Forster, D.G et al.; Org. Synth. Coll.; Vol.IV; 1964; S.771 folgendermaßen hergestellt werden.
Arylmagnesiumhalogenide werden mit elementarem Selen über die dabei entstehenden Polyselenide in Arylselenomagnesiumhalogenide überführt und diese dann hydrolysiert.

Erfindungsgemäß werden die in Anspruch 1 und Anspruch 2 unter anderem beschriebenen Selenosteroide nicht durch Umsetzung von Selenoarylen mit elektrophilen Steroidverbindungen erhalten, sondern Arylselenomagnesiumhalogenide werden in situ mit den entsprechenden steroidalen Elektrophilen, beispielsweise einem Oxiran oder Sulfonsäureester umgesetzt.
Diese Umsetzungen von Arylselenomagnesiumhalogeniden mit Steroiden erlauben eine breite Variation von Selenosubstituenten an den verschiedenen Positionen des Steroidmoleküls.

Aus der Literatur (Akhmedov,I.M. et al.; Zh. Org. Khim.; 14(4);1978; S.881 sind nur Reaktionen von Arylselenomagnesiumbromiden mit Epichlorhydrin bekannt.

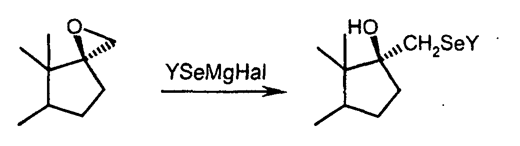

Gegenstand der vorliegenden Erfindung sind auch pharmazeutische Präparate zur oralen und parenteralen, incl. topischen, rektalen, subcutanen, intravenösen, intramuskulären, intraperitonealen, intranasalen, intravaginalen, intrabukkalen oder sublingualen Applikation, die neben üblichen Träger- und Verdünnungsmitteln eine im Anspruch 1 oder 2 aufgezeigte Verbindung als Wirkstoff enthalten.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die Vorteile der Erfindung ergeben sich im wesentlichen dadurch, daß neue Steroide mit radikophilen aromatischen Substituenten gefunden wurden,
- deren Wirkprofil sich von dem der bekannten Antioxidantien, einschließlich des Vitamin E unterscheidet,
- die als Wirkstoffe in pharmazeutischen Präparate zur Prophylaxe und Therapie radikalvermittelter Zellschädigungen eingesetzt werden können,
- die dabei im Vergleich zu herkömmlichen Präparaten eine hohe Wirksamkeit nachweisen

Die vorteilhafte Wirkung der erfindungsgemäßen Systeme als Inhibitoren der Lipidperoxidation und LDL-Oxidation werden in Tabelle 1 und Tabelle 2 demonstriert.

Als Vergleiche zu den erfindungsgemäßen Systemen dienten 17β-Estradiol, Estriol, U-78517F und Vitamin E, ebenfalls in Tabelle 1 und Tabelle 2 dargestellt.

Die Messung der In-vitro-Hemmwirkung auf die Lipidperoxidation wurde mittels des Thiobarbitursäuretestes vorgenommen.

Mit der Aussage zu den IC₅₀-Hemmwerten wird die lipidperoxidationshemmende Wirkung der jeweiligen Verbindung charakterisiert.
IC₅₀ gibt die Menge der zuzugebenden Substanz an, um eine 50%ige Hemmung der Lipidperoxidation zu erzielen (Tabelle 1).

Die Messung der In-vitro Hemmwirkung auf die LDL-Oxidation wurde nach ESTERBAUER et al. (1988): Effect of peroxidative conditions on human plasma low density lipoproteins. In: Eicosanoids, lipid peroxidation and cancer (Eds. Nigam et al.), S. 203-214, Springer-Verlag Berlin, Heidelberg, New York, vorgenommen.

Die in Tabelle 2 aufgeführten Werte für eine Hemmung der LDL-Oxidation stellen Beispiele für Herz-Kreislauf-Aktivitäten dar.

**Tabelle 1**

| Lipidperoxidationshemmung zu ausgewählten Verbindungen | |
|---|---|
| Verbindung | Lipidperoxidationshemmung [ IC₅₀ : µM ] |
| 17β-Estradiol | 12,4 |
| Estriol | 22,5 |
| Vitamin E | 132,0 |
| 6-3',5'-Dimethyl-4'-hydroxyphenyl-estra-1,3,5(10),6-tetraen-3,17β-diol | 0,95 |
| 17-4'-N,N-Dimethylaminophenyl-estra-1,3,5(10),16-tetraen-3-ol | 4,5 |
| 6-4'-N,N-Dimethylaminophenyl-estra-1,3,5(10),6-tetraen-3,17β-diol | 2,75 |
| 17α-4'-N,N-Dimethylaminophenyl-methyl-estra-1,3,5(10)-trien-3,17-diol | 3,53 |
| 17α-4'-N,N-Dimethylaminophenyl-methyl-estra-1,3,5(10),9(11)-tetraen-3,17-diol | 2,1 |
| 17α-4'-N,N-Dimethylaminophenyl-methyl-3-methoxy-estra-1,3,5(10)-trien-17-ol | 1,64 |
| 17α-4'-Hydroxyphenyl-methyl-estra-1,3,5(10)-trien-3,17-diol | 4,03 |
| 17α-4'-Hydroxyphenyl-methyl-estra-1,3,5(10),9(11)-tetraen-3,17-diol | 0,32 |
| 17α-4'-Hydroxyphenyl-mercaptomethyl-estra-1,3,5(10)-trien-3,17-diol | 2,2 |
| 17α-4'-Hydroxyphenyl-mercaptomethyl-estra-1,3,5(10),9(11)-tetraen-3,17-diol | 0,44 |
| 17α-4'-Hydroxyphenyl-mercaptomethyl-3-methoxy-estra-1,3,5(10)-trien-17-ol | 4,32 |
| 17α-4'-Hydroxyphenoxy-methyl-3-methoxy-estra-1,3,5(10)-trien-17-ol | 2,64 |
| 17α-3',5'-Dimethyl-4'-hydroxyphenyl-selenomethyl-3-methoxy-estra-1,3,5(10)-trien-17-ol | 1,97 |
| 17α-4'-N,N-Dimethylaminophenyl-selenomethyl-3-methoxy-estra-1,3,5(10)-trien-17-ol | 1,33 |
| 17α-3',5'-Dimethyl-4'-hydroxyphenyl-methyl-3-methoxy-estra-1,3,5(10)-trien-17-ol | 2,34 |
| 17α-3',5'-Di-tert.-butyl-4'-hydroxyphenyl-methyl-3-methoxy-estra-1,3,5(10)-trien-17-ol | 12,62 |
| 17α-3',5'-Di-tert.-butyl-4'-hydroxyphenyl-selenomethyl-3-methoxy-estra-1,3,5(10)-trien-17-ol | 4,97 |

**Tabelle 2**

| LDL-Oxidations-Hemmwirkungen ausgewählter Verbindungen (1 µM) | |
|---|---|
| Verbindung | LDL-Oxidationshemmwirkung (Verlängerung der lag-Phase in min) |
| 17β-Estradiol | 40 |
| Estriol | 10 |
| U-78517F | 20 |
| 17α-4'-Hydroxyphenyl-mercaptomethyl-estra-1,3,5(10),9(11)-tetraen-3,17-diol | 270 |
| 17α-4'-Hydroxyphenyl-methyl-estra-1,3,5(10),9(11)-tetraen-3,17-diol | 230 |
| 17α-4'-N,N-Dimethylaminophenyl-methyl-3-methoxy-estra-1,3,5(10)-trien-17-ol | 195 |
| 6-3',5'-Dimethyl-4'-hydroxyphenyl-estra-1,3,5(10),6-tetraen-3,17β-diol | 180 |
| 17α-4'-N,N-Dimethylaminophenyl-methyl-estra-1,3,5(10)-trien-3,17-diol | 170 |
| 17α-4'-Hydroxyphenyl-mercaptomethyl-esträ-1,3,5(10)-trien-3,17-diol | 120 |
| 17α-4'-Hydroxyphenyl-methyl-estra-1,3,5(10)-trien-3,17-diol | 125 |

Es ist festzustellen, daß die erfindungsgemäß ermittelten Verbindungen eine nachweislich hohe Lipidperoxidations- bzw. LDL-OxidationsHemmwirkung in vitro und damit verbunden die Eigenschaft, als Radikalfänger wirken zu können, besitzen. Diese wirken gleich oder besser als die diesbezüglich herkömmlichen Verbindungen 17β-Estradiol, Estriol, U-78517F und Vitamin E. Zur Erreichung der gleichen Hemmwirkung bzw. besseren Hemmwirkung wird weniger Substanz benötigt.

Die erfindungsgemäßen Präparate stellen sowohl Inhibitoren der Lipidperoxidation als auch Inhibitoren der LDL-Oxidation dar und sind deshalb geeignet zur Prophylaxe und Therapie radikalvermittelter Zellschädigungen wie beispielsweise beim spinalen Trauma, des ischämischen (thromboembolischen) Schlaganfalls, der Ischämie, des Organschadens in der Reperfusionphase nach- Transplantationen, den chronisch-degenerativen Erkrankungen des ZNS, der Senilen Demenz vom Alzheimer-Typ (SDAT), des Asthma, der muskulären Dystrophie und degenerativer neurologischer Krankheiten u.a. in Form von ZNS-Intoxikations- bzw. Degenerationszuständen.

Die erfindungsgemäßen Präparate erweisen sich ebenfalls als vorteilhaft zur Prophylaxe und Therapie solcher durch radikalvermittelte Zellschädigungen hervorgerufenen Erkrankungen, wie die multiple Sklerose, die Skin Graft Reaction, Akute Pankreatitis, Lebernekrosen (z.B. virale Hepatitis), der hämorrhagische, traumatische und septische Schock, Entzündungszustände, wie die osteo- oder rheumatoide Arthritis, adjuvante Arthritis, Arthrose, das nephrotische Syndrom (immunologisch), das systemische Lupus erythematosis, die Adriamycin-induzierte Herztoxizität und neuroprotectiven Hirntumoren.

Die erfindungsgemäßen Präparate eigenen sich auch zur Prophylaxe und Therapie derartiger durch radikalvermittelte Zellschädigungen hervorgerufenen Erkrankungen, wie allergische Reaktionen, die Atherosklerose, die Entzündung unter dermatologischen, inflammatorischen und psoriatischen Bedingungen, der Stress-induzierte Ulcer, Migräne, die maligne Hyperthermie, das hypoxische Syndrom, das ischämische Bowel-Syndrom und die Reduzierung der notwendigen Dosis bei der therapeutischen Anwendung radikalabbauender Enzyme, wie z.B. Superoxiddismutase und Catalase.

Weiterhin sind die erfindungsgemäßen Arzneimittel als antitumorale Wirksubstanzen einsetzbar und eignen sich für die prophylaktische und therapeutische Behandlung von Herz- und Kreislauf-Krankheitszuständen.

Die Erfindung wird durch die nachfolgenden Ausführungsbeispiele näher erläutert, wobei die zugehörigen Strukturformeln in Tabelle 3 aufgeführt sind. Die im Text hinter der jeweiligen Bezeichnung der Verbindung in Klammer gesetzte und unterstrichene Zahl gibt in der Tabelle den Hinweis auf die zugehörige Strukturformel.

### Beispiel 1

### 17α-Pentamethylphenyl-selenomethyl-3-methoxy-1,3,5(10)-estratrien-17-ol (1)

3,35mmol (1,0 g) 3-Methoxy-1,3,5(10)-estratrien-17(S)-spirooxiran werden bei Raumtemperatur unter Rühren in 5 ml trockenem THF gelöst und unter Argon zur auf ca. 15 °C abgekühlten frisch aus 12 mmol (0,30 g) Mg, 10 mmol (2,27 g) Brompentamethylbenzen und 22 ml THF bereitete Grignardlösung getropft. Unmittelbar danach werden 9 mmol (0,70 g) Selenpulver unter Rühren zugegeben. Die Reaktionsmischung erwärmt sich schwach und färbt sich allmählich kaffeebraun. Der Reaktionsansatz wird ca. 2 Stunden bei Raumtemperatur gehalten, dann in eisgekühlte Ammoniumchlorid-Lösung eingerührt, die Fällung mit Methyl-tert.-butylether extrahiert, der Extrakt über Natriumsulfat getrocknet und im Vakuum eingeengt.
Das ölige Rohprodukt reinigt man durch Säulenchromatographie an Kiselgel 60 (0,040-0,063 mm) unter Druck (Elution mit Toluen/n-Hexan 9:1) und anschließende Umkristallisation aus Aceton/Methanol unter Zusatz von Diisopropylether.
Ausbeute: 0,97 g (55,2% d. Th.); Fp.: 144-146 °C;
[α]_{D} ²⁰ + 37,5° (CHCl₃)

### Beispiel 2

### 17α-3',5'-Dimethyl-4'-hydroxyphenyl-methyl-3-methoxy-estra-1,3,5(10)-trien-17-ol (2)

3,35 mmol (1,0 g) 3-Methoxy-1,3,5(10)-estratrien-17(S)-spirooxiran werden bei Raumtemperatur unter Rühren in 5 ml trockenem THF gelöst und unter Argon zur auf ca. 40 °C warmen, frisch aus 12 mmol (0,30 g) Mg, 8,8 mmol (2,45 g) 4-Brom-2,6-dimethyl-trimethylsilyloxybenzen und 10 ml THF bereiteten Grignardlösung getropft. Der Reaktionsansatz wird ca. 4 Stunden bei 60-65 °C gerührt, danach wieder auf Raumtemperatur gebracht und die Reaktionsmischung in eisgekühlte Ammoniumchlorid-Lösung eingerührt. Die milchige, klebrige Fällung extrahiert man mit Methyl-tert.-butylether, trocknet den Extrakt über Natriumsulfat und engt im Vakuum ein. Es verbleiben 2,4 g öliges Rohprodukt.
Dieses wird in 20 ml Aceton gelöst und 1 ml 2n-HCI hinzugegeben. Nach ca. 2 Stunden wird die Reaktionsmischung in eisgekühlte NaHCO₃-Lösung eingerührt, die gelbe klebrige Fällung mit Methyl-tert.-butylether extrahiert, der Extrakt über Natriumsulfat getrocknet , im Vakuum eingeengt und das erhaltene Rohprodukt durch Säulenchromatographie an Kieselgel 60 (0,040-0,063 mm) unter Druck (Elution mit Toluen/Essigsäureethylether 9:1) und anschließender Umkristallisation aus Methanol unter Zusatz von Diisopropylether gereinigt. Ausbeute: 0,15 g (10,7% d. Th.); Fp.: 174-178 °C;
[α]_{D}²⁰ + 44° (CHCl₃)

### Beispiel 3

### 17α-4'-N,N-Dimethylaminophenyl-selenomethyl-3-methoxy-1,3,5(10)-estratrien-17-ol (3)

3,35 mmol (1,0 g) 3-Methoxy-1,3,5(10)-estratrien-17(S)-spirooxiran werden bei Raumtemperatur unter Rühren in 5 ml trockenem THF gelöst und unter Argon zur auf 13-15 °C abgekühlten, frisch aus 12 mmol (0,30 g) Mg, 9 mmol (1,8 g) 4-Brom-dimethyl-anilin und 15 ml THF bereiteten Grignardlösung getropft. Unmittelbar nach beendeter Zugabe des Oxirans werden 9 mmol (0,70 g) Selenpulver unter Rühren zugegeben, worauf die Reaktionsmischung sich leicht erwärmt und kaffeebraun färbt. Der Reaktionsansatz wird noch ca. 4 Stunden bei Raumtemperatur gehalten, dann in eisgekühlte Ammoniumchlorid-Lösung eingerührt, die Fällung mit Methyl-tert.-butylether extrahiert, der Extrakt über Natriumsulfat getrocknet und im Vakuum eingeengt.
Das ölige Rohprodukt reinigt man durch Säulenchromatographie an Kieselgel 60 (0,040-0,063 mm) unter Druck (Elution mit Toluen) und anschließender Umkristallisation aus Methanol unter Zusatz von Diisopropylether.
Ausbeute: 0,15 g (9% d. Th.); Fp.: 110-111 °C;
[α]_{D}²⁰ + 39° (CHCl₃)

### Beispiel 4

### 17α-4'-Hydroxyphenyl-methyl-1,3,5(10)-estratrien-3,17-diol (4)

4,22 mmol (1,2 g) 3-Hydroxy-1,3,5(10)-estratrien-17(S)-spirooxiran werden bei Raumtemperatur unter Rühren in 10 ml trockenem THF gelöst und unter Argon einer aus 28 mmol (0,68 g) Mg, 24 mmol (5,88 g) 4-Brom-trimethylsilyloxybenzen und 18 ml THF frischhergestellten Grignardlösung getropft. Nach ca. 2 Stunden Reaktion bei Raumtemperatur wird der Reaktionsansatz für weitere 3 Stunden bei 50-60 °C gerührt, später wieder auf Raumtemperatur gebracht und die Reaktionslösung in acetonische Salzsäure eingerührt, nach ca. 15 min mit Eiswasser versetzt, der Extrakt über Natriumsulfat getrocknet und im Vakuum eingeengt.
Das Rohprodukt reinigt man durch Säulenchromatographie an Kieselgel- Merck 60 (0,040-0,063 mm) unter Druck (Elution mit Toluen/Essigsäureethylester 9:1 bis 7:3) und anschließender Umkristallisation aus Methanol.
Ausbeute: 0,10 g (6,3% d. Th.); Fp.: 238-242 °C;

### Beispiel 5

### 17α-4'-N,N-Dimethylaminophenyl-methyl-1,3,5(10),9(11)-estratetraen-3,17-diol (5)

9,0 mmol (2,54 g) 3-Hydroxy-1,3,5(10),9(11)estratetraen-17(S)-spirooxiran werden bei Raumtemperatur unter Rühren in 15 ml trockenem THF gelöst und unter Argon ca 60 ml einer aus 10 mmol (1,46 g) Mg, 55 mmol (11,0 g) 4-Brom-N,N-dimethylanilin und 60 ml THF frisch hergestellten Grignardlösung zugetropft. Nach ca. 4-stündiger Reaktion bei Raumtemperatur wird der Reaktionsansatz in eisgekühlte verdünnte Ammoniumchlorid-Lösung eingerührt. Die klebrige Fällung extrahiert man mit Methyl-tert.-butylether und trocknet den Extrakt über Natriumsulfat. Nach dem Einengen im Vakuum verbleiben 9,0 g eines dunkelgrünen zähen Öls, welches durch Säulenchromatographie an Kieselgel-Merck 60 (0,040-0,063 mm) unter Druck (Elution mit Toluen/Essigsäureethylester 9:1) und anschließender Umkristallisation aus Methanol gereinigt wird.
Ausbeute: 1,25 g (34,4% d. Th.); Fp.: 130-132 °C;
[α]_{D} ²⁰ + 74° (CHCl₃)

### Beispiel 6

### 3,17β-Dihydroxy-17α-4'-N,N-dimethylaminophenyl-1,3,5(10)-estratrien (6)

Eine Suspension von 4,8 mmol (1,2 g) getrocknetem Ce-III-chlorid in 5 ml THF wird unter intensivem Rühren unter Argonatmosphäre mit 7,8 mmol (7,8 ml) einer aus 14,6 mmol (350 mg) Magnesium, 12 mmol (2,4 g) p-Brom-N,N-dimethyl-anilin und 12 ml trockenem THF frisch hergestellten Grignardverbindung bei -10 °C tropfenweise versetzt. Nach ca. 45 Minuten gibt man 1,4 mmol (380 mg) Estron zu, rührt 1 Stunde bei-5 bis 0 °C und läßt dann auf Raumtemperatur erwärmen. Der Ansatz wird in 50 ml gesättigter Ammoniumchloridlösung eingerührt. Die Phasen werden getrennt und die wäßrige Phase mit Methyl-tert.-butylether nachextrahiert. Die vereinigten organischen Phasen werden mit Wasser gewaschen, über Natriumsulfat getrocknet und unter Vakuum eingedampft.

Der Rückstand wird durch Flashchromatographie an 40 g Kieselgel (0,040 bis 0,063 mm; Eluent n-Hexan/Essigester 2 : 1 v/v) gereinigt und aus Aceton umkristallisiert. Man erhält 354 mg einer nahezu weißen kristallinen Substanz.
Fp.: 212 bis 220 °C.

### Beispiel 7

### 3-Hydroxy-17-4'-N,N-dimethylaminophenyl-1,3,5(10),16-estratetraen (7)

0,64 mmol (250mg) 3β, 17β-Dihydroxy-17-4'-N,N-dimethylaminophenyl-1,3,5(10)-estratrien werden in 25 ml Aceton bei 50 °C gelöst und nach Zugabe von 1,8 ml 2N Salzsäure 8 Stunden gerührt. Anschließend gibt man bei Raumtemperatur 20 ml gesättigte Natriumhydrogencarbonatlösung zu. Nach 30 Minuten dampft man das Aceton im Vakuum ab und extrahiert den wäßrigen Rückstand mit 60 ml Methyl-tert.-butylether.

Die organische Phase wird mit Wasser gewaschen, über Natriumsulfat getrocknet, filtriert und im Vakuum eingedampft. Der Rückstand wird aus Aceton/n-Hexan kristallisiert.
Ausbeute: 142 mg als kristalline Substanz; Fp.: 188-193 °C, Nadeln

### Beispiel 8

### 3, 17β-Dihydroxy-6-4'-N,N-dimethylaminophenyl-1,3,5(10),6-estratetraen (8)

Unter Argonatmosphäre und Rühren werden zu 3 mmol (0,74 g) getrocknetem mit 5 ml trockenem THF suspendierten Cer-III-chlorid 3,8 mmol (3,8 ml) einer aus 14,6 mmol (350 mg) Magnesium, 12 mmol (2,4 g) p-Brom-N,N-dimethylanilin und 12 ml trockenem THF frisch hergestellten Grignardlösung bei -10 °C zugegeben. Nach 15 Minuten setzt man 0,8 mmol (350 mg) 3,17-Bis-trimethylsilyloxy-6-oxo-1,3,5(10)-estratrien, gelöst in 5,6 ml trockenem THF zu. Man rührt 1 Stunde bei-5 °C. Nach Aufwärmen auf Raumtemperatur wird der Ansatz in 50 ml gesättigter Ammoniumchloridlösung eingerührt. Nach Phasentrennung wird mit gesättigter Ammoniumchloridlösung und Wasser gewaschen. Die wäßrigen Phasen werden mit Methyl-tert.-butylether nachextrahiert. Die vereinigten organischen Phasen versetzt man mit 0,5 ml 6N Salzsäure und rührt 1 Stunde bei Raumtemperatur. Es werden dann 50 ml Wasser zugegeben und die Phasen getrennt. Nach Einstellen des pH-Wertes der wäßrigen Phase auf 8 wird diese mit Methyl-tert.-butylether nachextrahiert. Die vereinigten organischen Phasen trocknet man über Natriumsulfat, filtriert und engt unter Vakuum ein.
Das Rohprodukt wird durch Säulenchromatographie an 16 g Kieselgel (0,040-0,063 mm; n-Hexan/Essigester 70:30 v/v) gereinigt und anschließend aus Aceton kristallisiert. Man erhält 113 mg einer weißen kristallinen Substanz; Fp.: 215-220 °C.

### Beispiel 9

### 3,17β-Dihydroxy-6-3',5'-dimethyl-4'-hydroxy-phenyl-1,3,5(10),6-estratetraen (9)

Unter Feuchtigkeitsausschluß und Argonatmosphäre werden zu 6 mmol (1,5 g) getrocknetem, mit 7 ml THF suspendiertem CeCl₃ 6mmol (6 ml) einer aus 12,1 mmol (295 mg) Magnesium, 10mmol (2,7 g) 1-Brom-3,5-dimethyl-4-trimethylsilyloxy-benzen und 13 ml THF frisch hergestellten Grignardlösung bei -10 °C zugetropft. Danach setzt man 1,4 mmol (600 mg) 3,17β-Bis-trimethylsilyloxy-6-oxo-1,3,5(10)-estratrien zu und läßt noch 2 Stunden bei -10 °C rühren.
Das Reaktionsgemisch wird anschließend mit 45 ml Methylenchlorid verdünnt, mit 20 ml 6N HCI versetzt und eine Stunde bei Raumtemperatur gerührt.
Nach Phasentrennung und Nachextraktion der wäßrigen Phase mit Methylenchlorid wird mit gesättigter Natriumhydrogencarbonatlösung und Wasser gewaschen, über Natriumsulfat getrocknet und unter Vakuum eingedampft. Das als braunes Öl erhaltene Rohprodukt wird durch Säulenchromatographie an Kieselgel (0,040-0,063 mm;
n-Hexan/Essigester 70:30 v/v) gereinigt und aus Methylenchlorid kristallisiert.
Ausbeute: 312 mg als gelbe kristalline Substanz. Fp.: 172-178 °C

### Untersuchungen zur pharmakologischen Wirksamkeit der Radikalscavenger-Substanzen

Die Testung der Substanzen auf die Hemmwirkung bezüglich der LipidPeroxidation wurde folgendermaßen durchgeführt:

### Reaktionsansatz

1 ml biologische Probe (cont. 0,1 mg Plasmamembranen) incl. Fenton's Reagenz und Drug.
Die 1 ml Gesamtvolumen teilen sich auf in 0,01 bis 0,02 ml synaptosomale Membranfraktion; 0,1 ml Eisen(II)-chlorid (2 mM); 0,1 ml Wasserstoffperoxid (2mM), auf 1 ml auffüllen mit 0,9 % NaCI (nicht PBS) und Ethanol oder DMSO als Vehicle des zu testenden Drugs.

### Prozedere

Der Reaktionsansatz wird 30 min bei 37 °C inkubiert, anschließend mit 2 ml Reagenz A abgestoppt und 10 min bei konstanten 80 °C inkubiert.
Nach Abkühlen in einem Eisbad (10 min) wird die Probe in einer Kühlzentrifuge zentrifugiert (1000 x g; 4 °C). Der Überstand wird (bis zu 2 h stabil) bei 535 nm gegen den Blindwert gemessen, der bis auf die Membranfraktion alle Reagenzien enthält.
Als Vergleichswert dient der Ansatz, der neben der Membranfraktion NaCl sowie im gegebenen Falle Vehicle mit gleichen Anteilen erhält.

### Zusammensetzung des Reagenz A

15 % (w/v) Trichloressigsäure (15 g); 0,375 % (w/v) Thiobarbitursäure (375 mg) ; 0,25 N HCI (2,11 ml konz. HCI)
in 100 ml wäßriger Lösung.

### Prüfsubstanzen

Die Prüfsubstanzen werden vorzugsweise in Ethanol als 20 millimolare Stammlösungen angesetzt und entsprechend verdünnt. Geprüft wird im Dosierungsbereich 1 - 150 µM.
In allen Versuchsansätzen wird eine entsprechende Standardsubstanz mitgeführt.

### Bewertungsparameter

- Dosis-Wirkungsanalyse der Prüfsubstanzen.
- Ermittlung der Lipidperoxidationshemmwerte mit mindestens fünf Substanzkonzentrationen im Hemmbereich 30 bis 70 %, bezogen auf die Testwerte ohne Substanzeffekt.

Die Testung der Substanzen auf die Hemmwirkung bezüglich der LDL-Oxidation nach der Methode von Esterbauer et al. (1988) wurde folgendermaßen ausgeführt:

### Reaktionsansatz

2 ml biologische Probe (cont. 0,5 mg LDL, isoliert aus humanem Vollblut, incl. 10 µM CuSO₄ sowie 1 bis 150 µM Testsubstanz und Ethanol als Vehicle des zu testenden Drugs) im zellfreien Medium PBS.

### Prozedere

Der Reaktionsansatz wird bei RT über einen Zeitraum von mindestens 8 h inkubiert und spektralphotometrisch (Absorptionsmaximum des oxidierten LDL liegt bei 234 nm) verfolgt. Entsprechend den bei der Meßwellenlänge von 234 nm registrierten Extinktionsveränderungen in Gegenwart bzw. Abwesenheit von Testsubstanzen bzw. in der Gegenüberstellung des nativen zum oxidierten LDL werden definitive Aussagen zur Beeinflussung des oxidierten LDL durch Testsubstanzwirkung ermöglicht.

### Prüfsubstanzen

Die Prüfsubstanzen werden vorzugsweise in Ethanol als 20 millimolare Stammlösungen angesetzt und entsprechend verdünnt. Geprüft wird im Dosierungsbereich 1 - 150 µM.
In allen Versuchsansätzen wird eine entsprechende Standardsubstanz mitgeführt.

### Bewertungsparameter

- Dosis-Wirkungsanalyse der Prüfsubstanzen.
- Ermittlung der LDL-Oxidations-Hemmwerte, ausgewiesen als Verzögerung der LDL-Oxidation in Form einer zeitlich verlängerten lag-Periode (min).

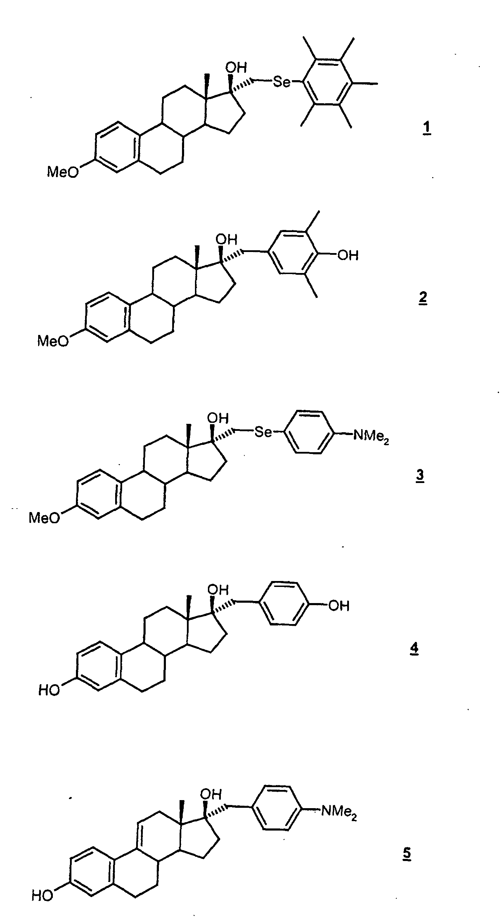

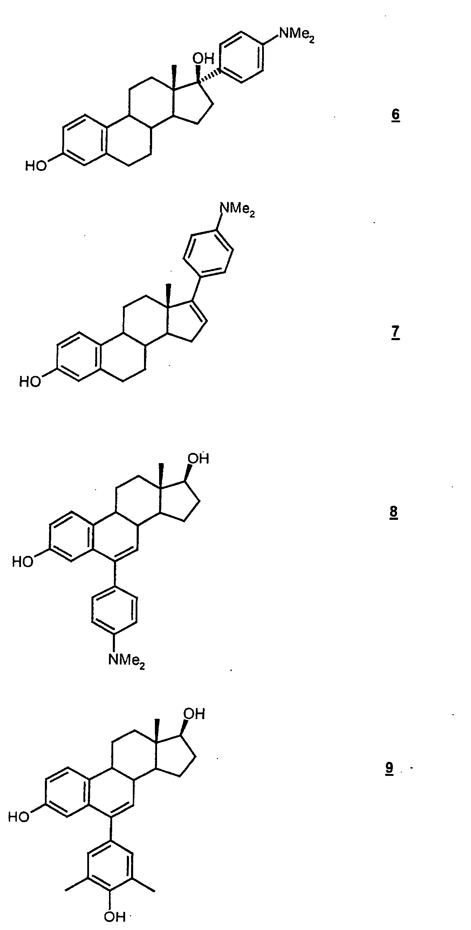

## Patentansprüche

1. Neue Steroide mit radikophilen aromatischen Substituenten der allgemeinen Formel I 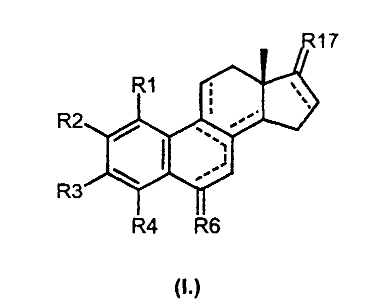 worin
R¹ bis R⁴ ein Wasserstoffatom,
eine Gruppierung OR', wobei R' ein Wasserstoffatom, eine Alkylgruppe mit jeweils ein bis vier C-Atomen und mindestens je ein R ein Wasserstoffatom und eine Gruppierung OR' darstellt
sowie
R⁶ für Wasserstoff steht oder
ein Substituent R⁶ eine Hydroxygruppe oder Teil einer 6,7-Doppelbindung und der andere eine Gruppierung -(CH₂)ₙY darstellt
mit n = 0 - 5
und 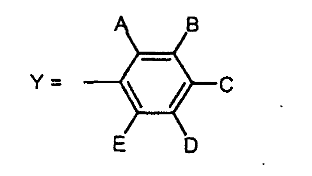 worin unter der Bedingung, daß mindestens einer der Substituenten eine Hydroxygruppe ist, A bis E unabhängig voneinander ein Wasserstoffatom,
eine Gruppierung OR' mit R' für ein Wasserstoffatom, eine Alkyl- oder Acylgruppe mit jeweils ein bis vier C-Atomen,
eine Alkylgruppe mit jeweils ein bis vier C-Atomen
oder
B, C, D eine Gruppierung -NR₂ mit R für eine Alkylgruppe mit jeweils ein bis vier C-Atomen und den jeweiligen vier anderen Substituenten Wasserstoff darstellen,
ferner
entweder
ein Substituent R¹⁷ eine β-Hydroxygruppe,
ein β-Wasserstoffatom oder Teil einer 16,17-Doppelbindung ist und der andere Substituent eine Guppierung -(CH₂)ₙX bezeichnet
mit
n = 0 - 1 für X = Y, OY, SY, SeY, NHY,
wobei Y die vorstehend angegebene Bedeutung hat
oder
ein Substituent R¹⁷ eine Gruppierung OR' mit R' für ein Wasserstoffatom, eine Alkyl- oder Acylgruppe mit jeweils ein bis vier C-Atomen oder Teil einer 16,17-Doppelbindung
und der andere ein Wasserstoffatom ist
oder
R¹⁷ eine Oxogruppe bedeutet und zusätzlich zum aromatischen Ring A das Steroidgerüst ein bis zwei Doppelbindungen als 6,7-; 7,8-; 8,9-; 9(11)-; 8(14)-; oder 16,17-Doppelbindungen aufweisen kann
unter Ausschluß der folgenden Substituentenkombination:
R¹ = R² = R⁴ = Wasserstoff
R³ = OCH₃
Einfachbindungen zwischen C7/C8, C8/C9, C8/C14 und C9/C11
ein Substituent R⁶ = Y und
ein Substituent R¹⁷ = H und der andere -OH
und unter der Bedingung, daß jedes Molekül der allgemeinen Formel I mindestens eine Gruppierung -Y, wie zuvor definiert, enthält.

2. Verbindungen nach Anspruch 1
nämlich
17-4'-N,N-Dimethylaminophenyl-estra-1,3,5(10),16-tetraen-3-ol
17α-4'-N,N-Dimethylaminophenyl-estra-1,3,5(10),-trien-3,17-diol
6-4'-N,N-Dimethylaminophenyl-estra-1,3,5(10),6-tetraen-3,17β-diol
17α-4'-N,N-Dimethylaminophenyl-methyl- estra-1,3,5(10)-trien-3,17-diol
17α-4'-N,N-Dimethylaminophenyl-methyl-estra-1,3,5(10),9(11)-tetraen-3,17-diol
17α-4'-N,N-Dimethylaminophenyl-methyl-3-methoxy-estra-1,3,5(10)-trien-17-ol
17α-4'-Hydroxyphenyl-methyl-estra-1,3,5(10)-trien-3,17-diol
17α-4'-Hydroxyphenyl-methyl-estra-1,3,5(10),9(11)-tetraen-3,17-diol
17α-4'-Hydroxyphenyl-mercaptomethyl-estra-1,3,5(10)-trien-3,17-diol
17α-4'-Hydroxyphenyl-mercaptomethyl-estra-1,3,5(10),9(11)-tetraen-3,17-diol
17α-4'-Hydroxyphenyl-mercaptomethyl-3-methoxy-estra-1,3,5(10)-trien-17-ol
17α-4'-Hydroxyphenoxy-methyl-3-methoxy-estra-1,3,5(10)-trien-17-ol
17α-3',5'-Dimethyl-4'-hydroxyphenyl-selenomethyl-3-methoxy-estra-1,3,5(10)-trien-17-ol
17α-4'-N,N-Dimethylaminophenyl-seleno-methyl-3-methoxy-estra-1,3,5(10)-trien-17-ol
17α-3',5'-Dimethyl-4'-hydroxyphenyl-methyl-3-methoxy-estra-1,3,5(10)-trien-17-ol
17α-3',5'-Di-tert.-butyl-4'-hydroxyphenyl-methyl-3-methoxy-estra-1,3,5(10)-trien-17-ol
und
17α-3',5'-Di-tert.butyl-4'-hydroxyphenyl-seleno-methyl-3-methoxy-estra-1,3,5(10)-trien-17-ol.

3. Verfahren zur Herstellung der Steroide nach den Ansprüchen 1 und 2, **dadurch gekennzeichnet**,
daß man die radikophilen Substituenten in Position 17 des Steroidgerüstes über die nucleophile Öffnung eines 17-Spirooxirans
oder direkt durch Alkylierung der 17-Oxogruppe einführt.

4. Verfahren zur Herstellung der Steroide nach den Anspruch 3 **dadurch gekennzeichnet, daß** man die direkte Alkylierung der 17-Oxogruppe über die Umsetzung entsprechender Metallorganyle mit Cer-III-Salzen durchführt.

5. Verfahren zur Herstellung von Selenosteroiden nach den Ansprüchen 1 oder 2 über die Umsetzung von Spirooxiranen und Epoxiden mit Arylselenomagnesiumhalogeniden, **gekennzeichnet dadurch**,
daß man Arylmagnesiumhalogenide
der allgemeinen Formel YMgHal mit elementarem Selen, in einem Ether zu den entsprechenden Arylselenomagnesiumhalogeniden umsetzt und diese in situ mit 17-Spirooxiranen oder 16,17α-, 15,16β-Epoxiden oder
5,10 α-Epoxiden zur Reaktion bringt.

6. Verfahren nach Anspruch 5, **dadurch gekennzeichnet,**
**daß** man die Arylmagnesiumhalogenide mit elementarem Selen in THF bei 0 bis 100 °C umsetzt.

7. Pharmazeutische Zusammensetzungen mit einem Gehalt an mindestens einer Verbindung nach Anspruch 1 oder 2.

8. Verwendung der Verbindungen nach einem
der Ansprüche 1 oder 2 zur Herstellung eines Arzneimittels zur Therapie und Prophylaxe von radikalvermittelten Zellschädigungen.

## Claims

1. Novel steroids having radicophilic aromatic substituents of the general formula I 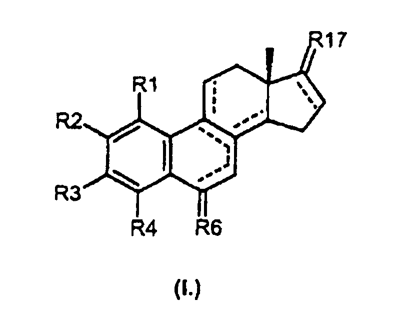 in which
R¹ to R⁴ is a hydrogen atom,
a group OR' where R' is a hydrogen atom, an alkyl group in each case having one to four C atoms and at least one R each is a hydrogen atom or a group OR'
and
R⁶ is hydrogen or
a substituent R⁶ of a hydroxyl group or part of a 6,7-double bond and the other is a group -(CH₂)ₙY where n = 0-5
and 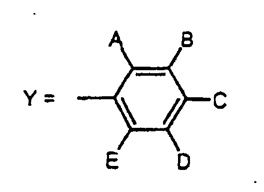 in which, with the condition that at least one of the substituents is a hydroxyl group, A to E independently of one another are
a hydrogen atom,
a group OR' where R' is a hydrogen atom, an alkyl or acyl group in each case having one to four C atoms,
an alkyl group in each case having one to four C atoms
or
B, C, D are a group -NR₂ where R is an alkyl group in each case having one to four C atoms and the respective four other substituents are hydrogen,
furthermore
either
a substituent R¹⁷ is a β-hydroxy group, a β-hydrogen atom or part of a 16,17-double bond and the other substituent is a group -(CH₂)ₙX
a substituent R¹⁷ is a β-hydroxy group, a β-hydrogen atom or part of a 16,17-double bond and the other substituent is a group -(CH₂)ₙX
where
n = 0-1 for X = Y, OY, SY, SeY, NHY,
where Y has the meaning indicated above
or
a substituent R¹⁷ is a group OR' where R' is a hydrogen atom, an alkyl or acyl group in each case having one to four C atoms or part of a 16,17-double bond
and the other is a hydrogen atom
or
R¹⁷ is an oxo group
and in addition to the aromatic ring A, the steroid structure can have one or two double bonds as 6,7-; 7,8-; 8,9-; 9(11)-; 8(14)-; or 16,17-double bonds
with the exclusion of the following substituent combination:
R¹ = R² = R⁴ = hydrogen
R³ = OCH₃
single bonds between C7/C8, C8/C9, C8/C14 and C9/C11
a substituent R⁶ = Y and
a substituent R¹⁷ = H and the other is -OH
and with the condition that each molecule of the general formula I contains at least one group -Y as defined beforehand.

2. Compounds according to Claim 1 namely
17-4'-N,N-dimethylaminophenyloestra-1,3,5(10), 16-tetraen-3-ol
17α-4'-N,N-dimethylaminophenyloestra-1,3,5(10),-triene-3,17-diol
6-4'-N,N-dimethylaminophenyloestra-1,3,5(10), 6-tetraene-3,17β-diol
17α-4'-N,N-dimethylaminophenylmethyloestra-1,3,5(10)-triene-3,17-diol
17α-4'-N,N-dimethylaminophenylmethyloestra-1,3,5(10),9(11)-tetraene-3,17-diol
17α-4'-N,N-dimethylaminophenylmethyl-3-methoxyoestra-1,3,5(10)-trien-17-ol
17α-4'-hydroxyphenylmethyloestra-1,3,5(10)-triene-3,17-diol
17α-4'-hydroxyphenylmethyloestra-1,3,5(10),9(11)-tetraene-3,17-diol
17α-4'-hydroxyphenylmercaptomethyloestra-1,3,5(10)-triene-3,17-diol
17α-4'-hydroxyphenylmercaptomethyloestra-1,3,5(10),9(11)-tetraene-3,17-diol
17α-4'-hydroxyphenylmercaptomethyl-3-methoxyoestra-1,3,5(10)-trien-17-ol
17α-4'-hydroxyphenoxymethyl-3-methoxyoestra-1,3,5(10)-trien-17-ol
17α-3',5'-dimethyl-4'-hydroxyphenylselenomethyl-3-methoxyoestra-1,3,5(10)-trien-17-ol
17α-4'-N,N-dimethylaminophenylselenomethyl-3-methoxyoestra-1,3,5(10)-trien-17-ol
17α-3',5'-dimethyl-4'-hydroxyphenylmethyl-3-methoxyoestra-1,3,5(10)-trien-17-ol
17α-3',5'-di-tert-butyl-4'-hydroxyphenylmethyl-3-methoxyoestra-1,3,5(10)-trien-17-ol
and
17α-3',5'-di-tert-butyl-4'-hydroxyphenylselenomethyl-3-methoxyoestra-1,3,5(10)-trien-17-ol.

3. Process for the preparation of the steroids according to Claims 1 and 2, **characterized in that** the radicophilic substituents in position 17 of the steroid structure are introduced via the nucleophilic opening of a 17-spirooxirane or directly by alkylation of the 17-oxo group.

4. Process for the preparation of the steroids according to Claim 3, **characterized in that** the direct alkylation of the 17-oxo group is carried out by means of the reaction of appropriate organometallic compounds with cerium(III) salts.

5. Process for the preparation of selenosteroids according to Claims 1 and 2 via the reaction of spirooxiranes and epoxides with arylselenomagnesium halides, **characterized in that** arylmagnesium halides of the general formula YMgHal are reacted with elemental selenium, in an ether, to give the corresponding arylselenomagnesium halides and these are reacted in situ with 17-spirooxiranes or 16,17α-, 15,16β-epoxides or 5,10α-epoxides.

6. Process according to Claim 5, **characterized in that** the arylmagnesium halides are reacted at 0 to 100°C in THF with elemental selenium.

7. Pharmaceutical compositions containing at least one compound according to Claim 1 or 2.

8. Use of the compounds according to either of Claims 1 and 2 for the production of a medicament for the therapy and prophylaxis of free radical-mediated cell damage.

## Revendications

1. Nouveaux stéroïdes ayant des substituants aromatiques radicophiles de formule générale I 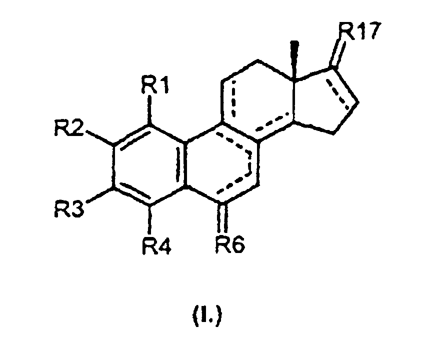 dans laquelle
R¹ à R⁴ représentent un atome d'hydrogène,
un groupe OR', dans lequel R' représente un atome d'hydrogène, un groupe alkyle ayant dans chaque cas un à quatre atomes de carbone et au moins un R représente chacun un atome d'hydrogène ou un groupe OR', et
R⁶ représente un hydrogène ou
un substituant R⁶ d'un groupe hydroxy ou une partie d'une double liaison en 6,7 et l'autre représente un groupe -(CH₂)ₙY
où n = 0 - 5
et 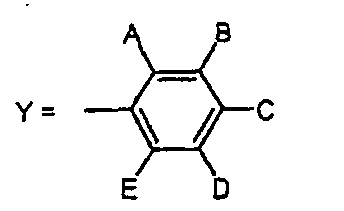 dans laquelle, à condition qu'au moins un des substituants représente un groupe hydroxy, A à E représentent, indépendamment les uns des autres,
un atome d'hydrogène,
un groupe OR' dans lequel R' représente un atome d'hydrogène, un groupe alkyle ou acyle ayant dans chaque cas de un à quatre atomes de carbone,
un groupe alkyle ayant dans chaque cas de un à quatre atomes de carbone,
ou
B, C, D représentent un groupe -NR₂ dans lequel R représente un groupe alkyle ayant dans chaque cas de un à quatre atomes de carbone et les quatre autres substituants respectifs représentent un hydrogène,
en outre,
soit
un substituant R¹⁷ représente un groupe hydroxy, un atome d'hydrogène en β ou une partie d'une double liaison en 16,17 et l'autre substituant représente un groupe -(CH₂)ₙX
dans lequel
n = 0 - 1 pour X = Y, OY, SY, SeY, NHY,
où Y a la signification indiquée ci-dessus
soit
un substituant R¹⁷ représente un groupe OR' dans lèquel R' représente un atome d'hydrogène, un groupe alkyle ou acyle ayant dans chaque cas de un à quatre atomes de carbone ou une partie d'une double liaison en 16,17,
et l'autre représente un atome d'hydrogène,
soit
R¹⁷ représente un groupe oxo,
et en plus du noyau aromatique A, la structure stéroïdique peut renfermer de un à deux doubles liaisons en tant que doubles liaisons en 6,7 ; 7,8, 8,9 ; 9(11) ; 8(14) ; ou 16, 17,
à l'exclusion des combinaisons de substituants suivantes :
R¹ = R² = R⁴ = hydrogène
R³ = OCH₃
liaisons simples entre C₇/C₈, C₈/C₉, C₈/C₁₄ et C₉/C₁₁
un substituant R⁶ = Y et
un substituant R¹⁷ = H et l'autre -OH
et à condition que chaque molécule de formule générale I renferme au moins un groupe -Y tel que défini ci-dessus.

2. Composés selon la revendication 1, à savoir
le 17-4'-N,N-diméthylaminophénylestra-1,3,5(10),16-tétraén-3-ol,
le 17α-4'-N,N-diméthylaminophénylestra-1,3,5(10)-triène-3,17-diol,
le 6-4'-N,N-diméthylaminophénylestra-1,3,5(10),6-tétraène-3,17β-diol,
le 17α-4'-N,N-diméthylaminophénylméthylestra-1,3,5(10)-triène-3,17-diol,
le 17α-4'-N,N-diméthylaminophénylméthylestra-1,3,5(10),9(11)-tétraène-3,17-diol,
le 17α-4'-N,N-diméthylaminophénylméthyl-3-méthoxyestra-1,3,5(10)-triène-17-ol,
le 17α-4'-hydroxyphénylméthylestra-1,3,5(10)-triène-3,17-diol,
le 17α-4'-hydroxyphénylméthylestra-1,3,5(10),9(11)-tétraène-3,17-diol,
le 17α-4'-hydroxyphénylmercaptométhylestra-1,3,5(10)-triène-3,17-diol,
le 17α-4'-hydroxyphénylmercaptométhylestra-1,3,5(10),9(11)-tétraène-3,17-diol,
le 17α-4'-hydroxyphénylmercaptométhyl-3-méthoxyestra-1,3,5(10)-trién-17-ol,
le 17α-4'-hydroxyphénoxyméthyl-3-méthoxyestra-1,3,5(10)-trién-17-ol,
le 17α-3',5'-diméthyl-4'-hydroxyphénylsélénométhyl-3-méthoxyestra-1,3,5(10)-trién-17-ol,
le 17α-4'-N,N-diméthylaminophénylsélénométhyl-3-méthoxyestra-1,3,5(10)-trién-17-ol,
le 17α-3',5'-diméthyl-4'-hydroxyphénylméthyl-3-méthoxyestra-1,3,5 (10)-trién-17-ol,
le 17α-3',5'-ditert-butyl-4'-hydroxyphénylméthyl-3-méthoxyestra-1,3,5(10)-trién-17-ol,
et
le 17α-3',5'-ditert-butyl-4'-hydroxyphénylsélénométhyl-3-méthoxyestra-1,3,5(10)-trién-17-ol.

3. Procédé de préparation des stéroïdes selon les revendications 1 et 2, **caractérisé en ce que** les substituants radicophiles en position 17 de la structure stéroïdique sont introduits par l'intermédiaire de l'ouverture nucléophile d'un 17-spiro-oxirane ou directement par alkylation du groupe 17-oxo.

4. Procédé de préparation des stéroïdes selon la revendication 3, **caractérisé en ce que** l'alkylation directe du groupe 17-oxo est réalisée par la réaction de composés organométalliques appropriés avec des sels de cérium(III).

5. Procédé de préparation de sélénostéroïdes selon les revendications 1 ou 2 par la réaction de spiro-oxiranes et d'époxydes avec des halogénures d'arylsélénomagnésium, **caractérisé en ce que** des halogénures d'arylmagnésium de formule générale YMgHal sont mis à réagir avec du sélénium élémentaire, dans un éther, pour donner lieu aux halogénures d'arylsélénomagnésium correspondants et ceux-ci sont mis à réagir in situ avec des 17-spiro-oxiranes ou des 16,17α-, 15,16β-époxydes ou des 5,10α-époxydes.

6. Procédé selon la revendication 5, **caractérisé en ce que** les halogénures d'arylmagnésium sont mis à réagir avec du sélénium élémentaire dans du THF entre 0 et 100°C.

7. Composition pharmaceutique contenant au moins un composé selon la revendication 1 ou 2.

8. Utilisation des composés selon l'une quelconque des revendications 1 ou 2 pour la préparation d'un médicament destiné à la thérapie et la prophylaxie de lésions cellulaires induites par des radicaux.
